# EUROPEAN PATENT APPLICATION

(11) **EP 3 865 157 A1**
(43) Date of publication of application: **18.08.2021**
(21) Application number: 20157717.8
(22) Date of filing: 17.02.2020
(51) Int. Cl.: A61M 1/00, A61B 17/32

(54) **FAT TISSUE REMOVAL DEVICE**

(71) Applicant: Université de Liège, 4000 Liège (BE); Centre Hospitalier Universitaire de Liège, 4000 Liège (BE)
(72) Inventor: Nikkels, Arjen, 4960 Malmedy (BE)
(74) Representative: V.O.

(57) **Abstract**

Fat tissue removal device comprising a cannula (100) comprising an evacuation tube (150) for connecting an evacuation system, a blunt cap (110) provided at a distal end of the cannula (100), and a rotatable shaft (130) comprising a cutting element (120) attached to the rotatable shaft, said rotatable shaft further being configured to be connectable to a driving device, the blunt cap (110) comprising a grinding chamber (160) for grinding fat tissue with the cutting element (120), an assembly comprising such device, a kit of parts for the removal of fat tissue, and a method for the removal of fat tissue with such device.

## Description

The present invention relates to a device for the removal of fat tissue from a subcutaneous region of a body and methods for removing fat tissue. Liposuction or suction lipectomy is an invasive, safe and effective method to eliminate localized deposits of subcutaneous fat tissue. In the early 1970's, the technique was developed using a blunt device that could be connected to a suction pump. A revolutionary improvement in the safety of these procedures was developed by Klein, introducing tumescent anesthesia. Since then, different devices have been developed in order to improve surgical and esthetic results with more or less success. The principle of the technique is, after the completion of the tumescent local anesthesia, to introduce a blunt needle into the fat tissue and to aspirate this fat tissue after mechanical disruption, using a back and forward movement. This movement sections the fat tissue while it is aspirated simultaneously like a cheese slicer. Several types of liposuction devices exist but they all require this back and forward movement. The problem with this cheese slicer motion is that it leads to the formation of subcutaneous canals and that it makes it difficult to perform classical lipoaspiration. Moreover, the existing devices do not enable to perform a precise and directed local lipoaspiration. For the same reasons, the devices are not adapted to remove lipomas, that are localized benign masses of the fat tissue, composed of hypertrophic adipocytes. Furthermore, this mandatory back and forward movement reduces the precision of the procedure, increases tissue traumatisms and the risk of bleeding.
It is known from US2002/0188280 A1 a fat removal device with a cannula having a closed tip that further includes a rotatable shaft and a blade attached to said shaft in order to cut fat tissue entering the cannula when said shaft is caused to rotate. However, such device necessitates to press the cannula against the mass of fat tissue. The internal blade only destroys the harvested fat to facilitate evacuation, but does not improve the direct harvesting of the fat tissue, nor its entry into the cannula. Operation of such device is therefore not optimal.
The present invention aims to solve or alleviate one or more of the above-mentioned problems. Particularly, the invention aims at providing a device permitting lipoaspiration in situ without the need of a back and forward movement. Particularly, the invention aims at providing a device that removes fat tissue in a subcutaneous region of a human body. Particularly the invention aims at grabbing fat tissue in a subcutaneous region of a human body, allowing harvested fat tissue to enter into the cannula, to be grinded and then evacuated.
To this aim, according to a first aspect, there is provided a fat tissue removal device comprising a cannula comprising an evacuation tube for connecting an evacuation system, a blunt cap provided at a distal end of the cannula, and a rotatable shaft comprising a cutting element attached to the rotatable shaft, said rotatable shaft further being configured to be connectable to a driving device; the blunt cap comprising a grinding chamber for grinding fat tissue with the cutting element, wherein the grinding chamber is formed by internal walls of the blunt cap and by an opening configured to allow entry of fat tissue inside the grinding chamber, the grinding chamber further being arranged to receive the rotatable shaft such that a portion of at least one tip of the cutting element extends out of said grinding chamber through said opening when in use in order to collect fat tissue inside said grinding chamber; and wherein the grinding chamber of the blunt cap is further configured to be connected to the evacuation tube to evacuate the grinded fat tissue.With the device and in particular with the at least one tip of the cutting element extending out of said grinding chamber, fat tissue is grabbed when it comes into contact with the cannula. This has the advantage to facilitate its entry or its uptake into the cannula and to avoid unnecessary movement of the cannula in the body.

Furthermore, the removal of fat tissue is performed through a grinding chamber, wherein the fat tissue is grinded and then evacuated through a tube to a reservoir. An advantage is that through grinding of the material, the evacuation of the fat tissue is facilitated, hence allowing a lesser diameter for the evacuation tube and consequently a smaller, more precise and easier to handle device. The device is suitable for the precise removal of localized fat tissue, such as lipomas, as well as for the removal of larger volumes of fat.
Such device enables the removal of fat tissue without the need for back and forth movement and without the need of pressing the cannula against a mass of fat tissue. After its entry, fat tissue may be cut through the rotatable cutting element and hence be removed easily. Hence the same blade allows the precise removal of the fat tissue as well as the grinding action of the fat tissue. The device enables the removal of fat tissue with precision and on a specific location without the need of a large incision, required for example with regular surgical removal of lipomas. The device is particularly suitable for the removal of fat tissue in a subcutaneous region of the human body. It is advantageously manipulated by the surgeon or doctor such that it avoids the injury of other tissue during the entry in the body and during action. The blunt cap permits to be advanced in the subcutaneous tissue without traumatism. Furthermore, as the cutting element may be not rotating until actioned at the requested site, there is only minimal risk of tissue injury of the surrounding tissues, blood vessels, etc.
The rotatable shaft and the cutting element may form two pieces that are attached to one another or fixed to one another. Advantageously the rotatable shaft and the cutting element may be manufactured in one piece. Advantageously the rotatable cutting element is made of stainless steel, which is a material that can be sterilized easily.

Advantageously, the rotatable shaft may be connected to a driving device during operation and may be separated after the end of operation. This allows the easy cleaning and sterilization of the device. Preferably the driving device enables to work with a variable speed of rotation. Preferably, the rotation speed can be selected to be in the range from 0 to 15 000 t/min. Advantageously during action of the driving device, there is no warming-up of the motor. Preferably, the driving device does not cause any vibration nor heating during action.
Advantageously the connection of the evacuation tube with the grinding chamber may be positioned at the back of the grinding chamber allowing the fat tissue to be grinded before being evacuated. This may avoid to evacuate fat tissue through the evacuation tube that has not been grinded before. Hence it avoids clogging in the evacuation tube and eventually in the evacuation system connected to said evacuation tube.
The evacuation system is preferably configured such that there is no clogging occurring in the system during operation. Advantageously, the device enables a narrow diameter of cannula to be used thanks to the grinding action of the cutting element on said fat tissue.
The opening of the fat tissue removal device may advantageously be a lateral opening with respect to the rotatable shaft. In such case, the blunt cap of the device may comprise internal side walls, a top and a bottom wall, and the lateral opening is in a side wall of the blunt cap. The opening may be lateral with respect to the longitudinal axis of the cannula, which corresponds to the axis of the rotatable shaft. The lateral opening with respect to the rotatable shaft advantageously helps the grabbed fat tissue to enter and stay inside the grinding chamber. The rotation movement of the cutting element inside the grinding chamber may further help in bringing the fat tissue inside the chamber. In fact, thanks to this arrangement, the tip of the cutting element, while rotating, exits the chamber and returns inside the chamber, thereby facilitating the grabbing of tissue outside the chamber and bringing of tissue inside the chamber.
The centre of the cutting element of the device may be connected to the rotatable shaft slantwise. Advantageously, the cutting element is inclined on the shaft, forming at least one acute angle between the cutting element and the rotatable shaft. The at least one acute angle is less than 90°. More preferably the at least one acute angle is between 15° and 85°.
The cutting element of the device may comprise at least one blade extending from the rotatable shaft. When the cutting element is formed of at least one blade, the device offers a more selective cutting of fat tissue versus other types of tissue such as for example fibrous tissue. This is particularly suitable for the removal of lipomas.
The at least one blade of the device may be arc-shaped, preferably S-shaped. When the cutting element is formed of an at least arc-shaped blade, the cutting surface may be increased and the efficacy of cutting and grinding may be improved. The blade may be curved in a plane or the blade may be curved helicoidally. When the cutting element is formed of at least one blade that forms an S-shape, the efficacy of cutting is further improved. Advantageously, the choice of the form of the cutting element is combined with a suitable speed of rotation of the rotatable shaft to enable a grinding action of fat tissue.
The cutting element may be arranged substantially diagonally with respect to the grinding chamber such that during use it occupies the majority of the grinding chamber volume. Advantageously, the length of the at least one blade is close to at least one dimension of the opening of the blunt cap. Even more preferably, the length of the at least one blade and the at least one acute angle formed between the at least one blade and the rotatable shaft are together such that a volume occupied by the at least one blade during rotation is as close as possible to the volume of the grinding chamber. This has the advantage that no fat tissue gets stuck or accumulates inside the chamber, or at the chamber corners. Fat tissue that has entered the chamber gets cut, grinded and can be evacuated easily through the cannula. Preferably at least one tip of the cutting element is sharp. More preferably all tips of the blade are sharp in order to improve the cutting effect of the device.
The rotatable shaft of the device may advantageously be off-centred with respect to the centre of the grinding chamber. When the rotatable shaft is off-centred in the device with respect to the centre of the grinding chamber, the cutting element connected to said shaft is itself off-centred. Advantageously, when the opening of the grinding chamber is a lateral opening, the rotatable shaft may be closer to said opening, such that at least a portion of the cutting element may be located closer to said opening. This ensures that a portion of the cutting element extends out of the lateral opening during rotation, which improves the grabbing of fat tissue outside the chamber. This facilitates the cutting of fat tissue entering the chamber. When the rotatable shaft is off-centred towards the opening, the evacuation tube is preferably centred or off-centred towards the back of the grinding chamber, allowing the space in the back to evacuate the grinded fat tissue by the evacuation tube.
When the opening for entry of fat tissue of the blunt cap is an opening in the top wall of said blunt cap, the rotatable shaft may be central with respect to the longitudinal central axis of the cannula. If it is off-centred with respect to the centre of the grinding chamber towards the opening, at least a portion of the cutting element extends out of said grinding chamber to grab fat tissue, which is then evacuated by means of the evacuation tube.
The blunt cap of the device may advantageously be detachable from the cannula, and/or the shaft tube and/or the evacuation tube. This enables an easier cleaning or sterilizing of the device. Advantageously, the blunt cap may be disposable while the rest of the cannula may be cleaned or sterilized. The blunt cap and the shaft tube may also form one single piece. The blunt cap and the evacuation tube may also form one single piece. The blunt cap, the shaft tube and the evacuation tube may form one single piece. In a particular case, the shaft tube may be included in the evacuation tube. Advantageously, the means to attach the blunt cap provide a secure system which avoids the blunt cap to detach itself during a procedure.
The blunt cap may further comprise a second opening located along the axis of the rotatable shaft for the insertion of said rotatable shaft. The axis of the rotatable shaft coincides with the longitudinal axis of the cannula. Such second opening is preferably located in a top wall of the blunt cap. When the device further comprises such second opening, the device may be more easily assembled. For example, the cutting element and the rotatable shaft, when forming one piece, may be easily inserted through this second opening.
The width size of the walls of the blunt cap may preferably be less than 20 mm, preferably less than 10 mm. Advantageously a reduced size of the device enables a reduced cut or incision of the skin of the patient to enter the device and hence the scar left in the skin of the patient will be reduced. The device may have such a length that it enables the incision line to be at a distance from the actual targeted fat lesion, in order to hide or to reduce the visibility of the scar.
The blunt cap may preferably be made of plastic or ceramic or stainless steel. Preferably the material of the grinding chamber is made of a material that is not expensive. Preferably the grinding chamber can be printed with a 3D printer. This enables to adapt the form of the chamber easily. The chamber may be sterilized with the rest of the device before use or the chamber may be a disposable piece that is fixed on the device before use.

The rotatable shaft may further be provided inside a shaft tube and/or be provided inside the evacuation tube. Preferably, the rotatable shaft is provided inside a shaft tube so that the rotatable axis of the shaft is separated from the evacuation of grinded fat tissue. This also reduces the risk of clogging in the evacuation tube. The use of a cannula with a set of two tubes (evacuation tube and shaft tube) is also advantageous in that it may reduce or eliminate vibration due to the rotation of the driving device when in use.
The fat tissue removal device may further comprise a housing configured to enclose at least partly the shaft tube and the evacuation tube. A housing may advantageously be used to protect the shaft tube and the evacuation tube and to allow the device to be hand-hold. Preferably the housing is not circular, which enables to avoid any vibration. Advantageously the housing is detachable from the device to enable an easier cleaning or sterilizing of the device. The housing may be in any material. Preferably the housing is in plastic while the shaft tube and the evacuation tube are in stainless steel. According to another aspect, there is provided a fat tissue removal assembly comprising a fat tissue removal device and an evacuation system comprising an evacuation conduit and a vacuum pump, said evacuation conduit being connected to said fat tissue removal device and being configured to evacuate fat tissue. Advantageously, the evacuation system provided in the assembly is such that there is no clogging in the grinding chamber. The evacuation system may further comprise a fat tissue disposal reservoir. Advantageously, the evacuation system is configured such that pumping or not can be decided during action.
According to yet another aspect, there is provided a kit of parts for the removal of fat tissue comprising a blunt cap configured to be fixed to a cannula comprising an evacuation tube connected to an evacuation system and a rotatable shaft comprising a cutting element attached to the rotatable shaft, said rotatable shaft being configured to be driven by a driving device; wherein the blunt cap comprises a grinding chamber for grinding fat tissue with the cutting element; wherein the grinding chamber is formed by internal walls of the blunt cap and by an opening configured to allow entry of fat tissue inside the grinding chamber; wherein the grinding chamber is further arranged to receive the rotatable shaft such that a portion of at least one tip of the cutting element extends out of said grinding chamber through said opening when in use in order to collect fat tissue inside said grinding chamber; and wherein the grinding chamber of the blunt cap is further configured to be connected to the evacuation tube to evacuate grinded fat tissue.
Such a kit of parts can be used to easily retrofit existing systems for the removal of fat tissue, which are already equipped with an evacuation system for example.

According to yet another aspect, there is provided a method for the removal of fat tissue comprising the steps of inserting the cannula of the fat tissue removal device or of the fat tissue assembly, in a fat tissue region of a body; activating the rotatable shaft of said fat tissue removal device and optionally activating the evacuation device; grabbing fat tissue from the fat tissue region of the body through the opening of the blunt cap of the cannula; cutting said fat tissue in the grinding chamber of said fat tissue removal device; removing said grinded fat tissue through the evacuation tube.
The method enables an easy entry of fat tissue into the cannula and an optimal removal of fat tissue through a grinding action that is obtained by the cutting element and the rotatable shaft. Advantageously, the method does not require any injection of liquid such as for example water.
There is also provided a method of retrofitting a fat tissue removal device comprising a cannula by providing the blunt cap and the rotatable shaft of the kit of parts to the cannula of the device. It is an advantage that an existing system can easily be improved simply by providing the rotatable shaft and the blunt cap.

The present invention will be further described with reference to figures of exemplary embodiments. Corresponding elements are designated with corresponding reference signs.
Figure 1 is a general view of a device.
Figure 2 is a cross section of the distal end of a device.
Figure 3 is a front view of the distal end of a device.
Figure 4 is a top view of the distal end of the device shown in Figure 3.
Figure 5 is a detailed view of an example of a device.
Figure 6 is a side view of the distal end of a device.
Figure 7 is an image showing different types of cutting elements.

### Definitions

By a cannula, one means a tube or a set of two tubes, also referred to as twin tubes, that can be inserted into a human body through an incision of the skin.

By a blunt cap, one means a cap at the distal end of a cannula that has rounded-off corners. It may be the extension of the tube or of the twin tube or it may be a cap that is fixed to the distal end of the cannula.

By an evacuation tube, one means a tube that is part of the cannula and that is configured to evacuate matter, in particular fat tissue. It may be a rigid tube or it may be a soft tube. Preferably it is a rigid tube. Preferably it is coupled to a shaft tube.

By a shaft tube, one means a tube that is part of the cannula and that is configured to be connectable to a driving device and that may protect a rotatable shaft. The shaft tube may be the proximal end of the cannula. In some cases, the shaft tube and the evacuation tube are connected or even merged to form one single tube. In other cases, the shaft tube is included in the evacuation tube. In yet other cases, the rotatable shaft is directly connected to a driving device without the need for a shaft tube.

The terms "about" or "approximate" and the like are synonymous and are used to indicate that the value modified by the term has an understood range associated with it, where the range can be +20%, +15%, +10%, +5%, or +1%. The term "substantially" is used to indicate that a result (e.g., measurement value) is close to a targeted value, where close can mean, for example, the result is within 80% of the value, within 90% of the value, within 95% of the value, or within 99% of the value.

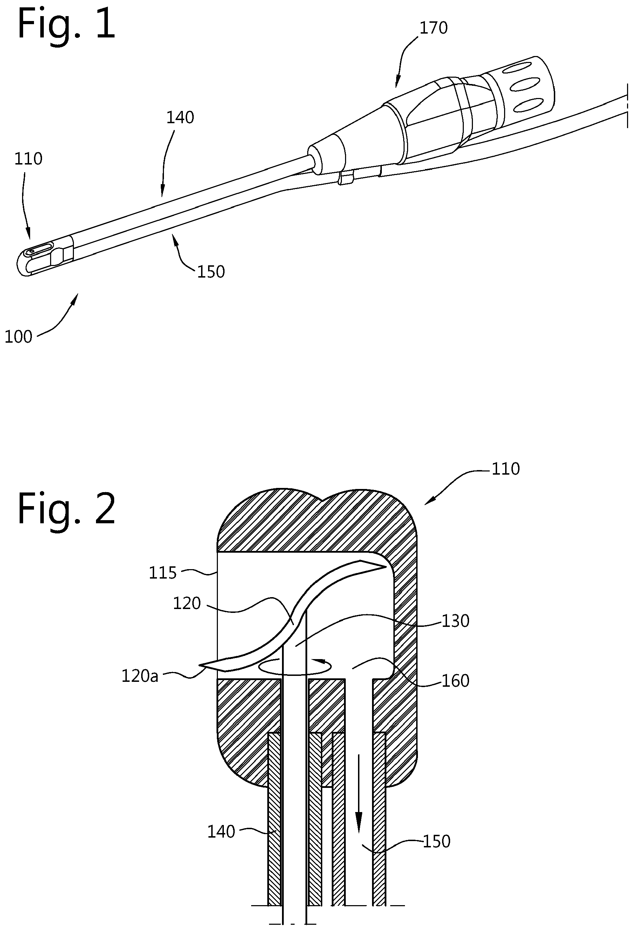

Figure 1 shows an example of a device for removing fat tissue wherein a cannula 100 is connected to a driving device 170 that can be held in hand.

The cannula 100 can comprise a shaft tube 140, which comprises a rotatable shaft connected to the driving device and an evacuation tube 150 connected to an evacuation system (not shown). The cannula has a blunt cap 110 that is covering both the shaft tube and the evacuation tube. The cap is blunt such that the cannula enters a subcutaneous region of the body and reach a fat tissue without damaging the surrounding tissues. The blunt cap has preferably a lateral opening 115 for allowing the entry of fat tissue into the cannula.

Figure 2 shows a cross section of a distal end of a cannula. The distal end can comprise the blunt cap 110 of the cannula with a lateral opening 115 in said cap for allowing the entry of fat tissue into the cannula. The blunt cap 110 is preferably hollow so that is forms a grinding chamber 160, delimitated by the internal walls of the cap. The internal walls may comprise a bottom wall, lateral walls and a top wall at the top of the cap.

The blunt cap is preferably attached to the shaft tube and to the evacuation tube, both tubes being preferably parallel one to another.

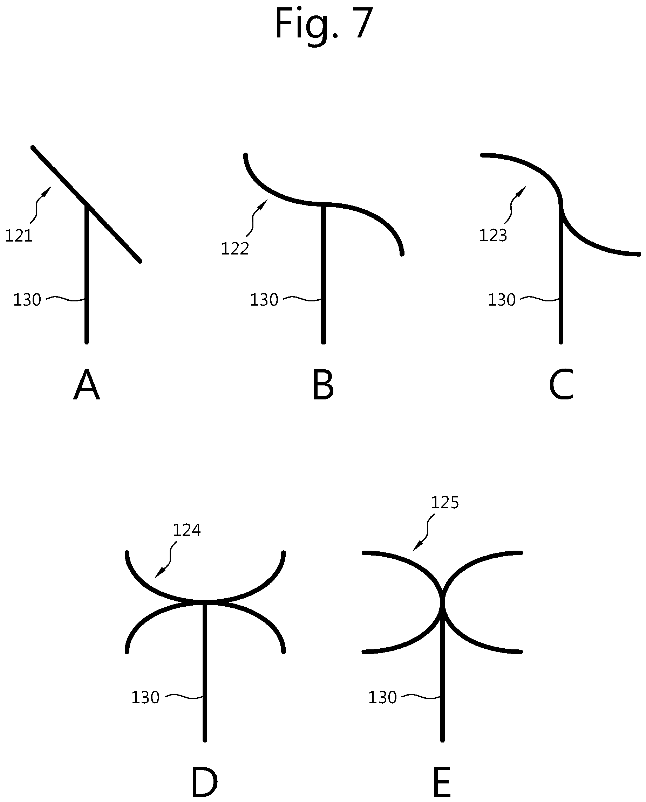

A cutting element 120 is at least partly located in the grinding chamber 160 and fixed onto a rotatable shaft 130. The rotatable shaft can be inserted in the shaft tube 140. In the present example, the cutting element is a blade having an S-shape. Other shapes are also possible as described hereunder in reference to Figure 7.

The blade 120 can be fixed to the shaft by its centre and can be positioned slantwise on the shaft so that the blade occupies most of the volume of the grinding chamber when in rotation. Preferably, the blade 120 is arranged diagonally with respect to the grinding chamber such that during rotation of the shaft, the tip of the blade comes as close as possible to the corners of the chamber. This also allows to avoid fat tissue to accumulate in the corners of the grinding chamber. The diagonal arrangement also allows to provide the maximal cutting surface within the chamber.

Therefore, the dimensions of the blade 120 depend on the dimensions of the grinding chamber 160 in order to optimize the grinding process of the fat tissue inside the chamber. Therefore, the angle formed by the shaft and the cutting element also depends on the volume of the chamber.

As illustrated in Figure 2 for example, a tip 120a of the blade 120 is extending out of the grinding chamber through the opening 115, thereby allowing for an easy uptake of fat tissue during operation. The rotating action of the driving device enables the tip 120a of the cutting element 120 that is extending out of the grinding chamber 160 to grab the fat tissue in proximity of the cannula. In addition, fat tissue may be pushed manually by the doctor holding the device towards the opening of the blunt cap of the cannula to further help the aspiration process. Rotation movement of the cutting element induces the fat tissue to be brought inside the grinding chamber 160 where the continuous rotation will grind such fat tissue. An aspiration action of an evacuation device connected to the device may also suck the fat tissue towards the opening. Grinded fat tissue may then be aspirated by the evacuation device. Preferably, the different actions are combined for a better result.

Preferably, substantially 5 to 30 %, even more preferably 10 to 20 % of the cutting element extends out of the chamber during rotation, as illustrated for example in Figure 2, with the tip of the blade 120a extending out of the chamber 160.

Preferably, the tips 120a of the blade are sharp such that it grabs the fat tissue more easily. This also improves the grinding of the fat tissue inside the chamber 160. The tips of the blade can even have slightly a hook shape such that the fat tissue attaches its self to the blade before entering the chamber.

The rotatable shaft is preferably off-centred in the cannula with respect to the longitudinal central axis of the cannula. This also allows the tip of the blade to extend out of the chamber through the lateral opening 115 during use.

The evacuation tube 150 is preferably connected to the grinding chamber 160 so that is allows for evacuation of fat tissue after grinding. The position of the evacuation tube connection at the back of the grinding chamber (in the bottom wall) is particularly suitable for the evacuation of fat tissue without clogging. When the device is in operation, the fat tissue gets grabbed by the tip 120a of the cutting element 120, is then grinded and is lastly evacuated. Evacuation is performed by connecting the evacuation tube to a vacuum device.

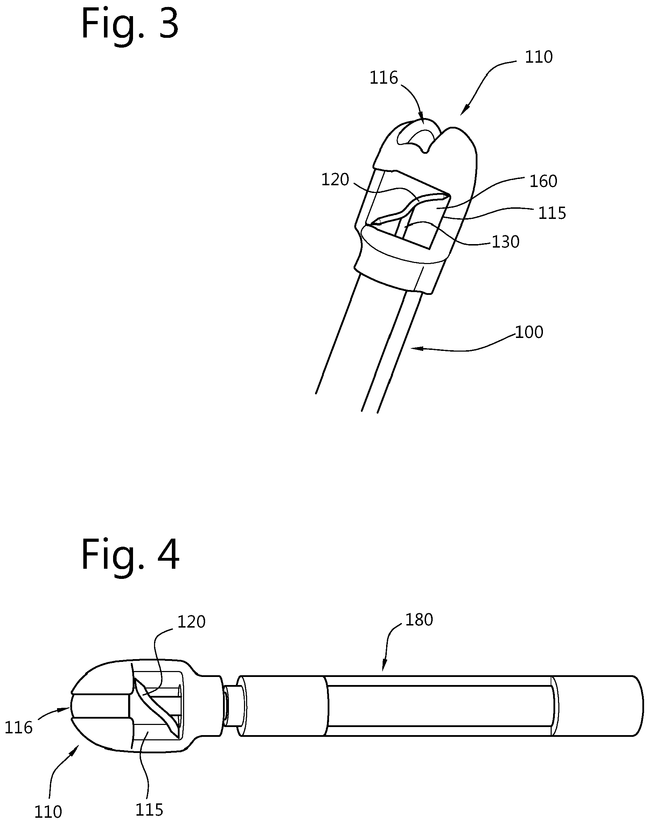

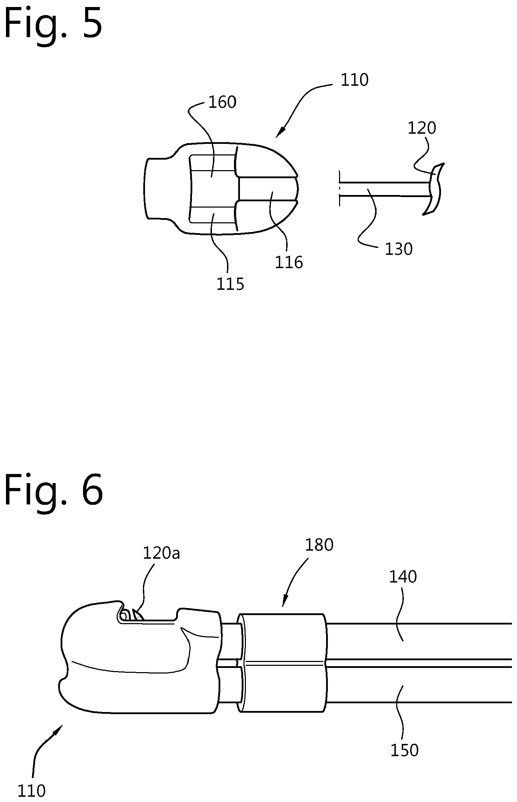

Figure 3 is a front view in three dimensions of the distal end of another example of a cannula for removing fat tissue, wherein the distal end comprises a blunt cap 110 with a lateral opening 115 for the entry of fat tissue. A grinding chamber 160 is formed in the blunt cap and is delimitated by the internal walls of the blunt cap, the internal walls comprising a top wall, a bottom wall and side walls. In this grinding chamber is provided a cutting element 120 attached to a rotatable shaft 130. The rotatable shaft 130 is connected to the chamber by means of the bottom wall. In this example, the blunt cap further comprises a second opening 116 located in the top wall, for allowing an easy dismantling of the device. The rotatable shaft can be dismantled from the cannula by passing through this second opening as shown in Figure 5. Therefore, the length of the opening 116 depends as well on the dimensions of the blade 120 such that it can easily pass through it.

Figure 4 is a top view of the cannula for removing fat tissue shown in Figure 3 wherein the distal end comprises a blunt cap 110, a shaft tube 140 and an evacuation tube (not visible). The blunt cap has two openings, one lateral opening 115 for allowing the entry of fat tissue and one top opening 116 for allowing the dismantling of the rotatable shaft and its blade that is located in the shaft tube. A cutting element in the form of an S-shaped blade is attached to the rotatable shaft. In this example, the cannula further comprises a protective housing 180 that allows for a better manipulation of the device with the hand. The protective housing is surrounding at least part of the shaft tube and of the evacuation tube, both tubes being coupled.

Figure 5 is a detailed view of the blunt cap 110 of the cannula when the rotatable shaft 130 has been dismantled from the blunt cap and from the cannula, by passing through the top opening 116.

Figure 6 is a side view of the distal end of the cannula 100 illustrated in the examples of Figures 3, 4 and 5. The distal end comprises a blunt cap 110 with a lateral opening 115. This side view also shows the tip 120a of the cutting element 120, which is a blade in this example, that is extending out of the grinding chamber 160 through the lateral opening 115 in the blunt cap. The distal end can also comprise a shaft tube 140, attached to the tip such that the tip of the blade 120a extends out of the grinding chamber, and an evacuation tube 150 attached such that it is in the back of the grinding chamber, as seen from this Figure. The shaft tube is closer to the lateral opening of the blunt cap and the evacuation tube is further away from the lateral opening. Both tubes are preferably attached together by means of a housing 180 to solidify the device.

Figure 7 shows different examples of cutting elements. In the example of Figure 7a, the cutting element is a straight blade oriented slantwise to the rotatable shaft. In another example, Figure 7b, the cutting element is an S-shaped blade oriented slantwise to the rotatable shaft. In a third example,

Figure 7c, the cutting element is again an S-shaped, however oriented differently from the previous one. In the examples of Figures 7d and 7e, the cutting element is a double blade forming a curved X-shape. Beside these examples, the cutting element or the at least one blade can have many different shapes. In a preferred example, the cutting element may be terminated by one or more hooks configured to grab the fat tissue. Preferably, the shape of the cutting element should be such that it maximizes the cutting surface within the grinding chamber, without obstructing the passage of fat tissue, while enabling an optimal grinding efficiency of the fat tissue, and while avoiding clogging of the destroyed and grinded fat tissue. For example, blades with substantially helicoidal shapes which fulfil these requirements could also be considered.

In one example, the blunt cap has the following dimensions: an external length of substantially 12mm and an external width of substantially 12mm. The opening length and width of the blunt cap allowing for entry of fat tissue are respectively 10mm and 6mm. Larger and smaller dimensions of the grinding chamber are possible, according to requested range of clinical use.

In one example, the blade has a length of 8.5mm and a width of 1mm. Thicker and/or larger variations are possible alternative options. As indicated earlier, the size of the blade should be matched with the size of the grinding chamber.

The rotation speed can preferably be set in the range of 0 to 15 000 t/min. A suitable rotation speed of a driving device is 1000-1200 t/min. Speed range of from 0 t/min to 1000-1200 t/min are preferred, in order to activate and modulate rotation and grinding action when desired.

It should be clear to the person skilled in the art that the invention is not limited to the embodiments described above. Many alternatives are possible within the scope of protection as formulated in the claims hereafter.

For the purpose of clarity and a concise description, features are described herein as part of the same or separate embodiments, however, it will be appreciated that the scope of the invention may include embodiments having combinations of all or some of the features described. It may be understood that the embodiments shown have the same or similar components, apart from where they are described as being different.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage. Many variants will be apparent to the person skilled in the art. All variants are understood to be comprised within the scope of the invention defined in the following claims.

## Claims

1. Fat tissue removal device comprising:
- a cannula (100) comprising
- an evacuation tube (150) for connecting an evacuation system,
- a blunt cap (110) provided at a distal end of the cannula (100), and
- a rotatable shaft (130) comprising a cutting element (120) attached to the rotatable shaft, said rotatable shaft further being configured to be connectable to a driving device;
the blunt cap (110) comprising a grinding chamber (160) for grinding fat tissue with the cutting element (120);
wherein the grinding chamber is formed by internal walls of the blunt cap (110) and by an opening (115) configured to allow entry of fat tissue inside the grinding chamber, the grinding chamber further being arranged to receive the rotatable shaft such that a portion of at least one tip of the cutting element (120) extends out of said grinding chamber (160) through said opening when in use, in order to collect fat tissue inside said grinding chamber; and wherein the grinding chamber of the blunt cap (110) is further configured to be connected to the evacuation tube (150) to evacuate grinded fat tissue.

2. Fat tissue removal device according to claim 1, wherein the opening (115) is a lateral opening with respect to the rotatable shaft (130).

3. Fat tissue removal device according to claim 1 or 2, wherein the centre of the cutting element (120) is connected to the rotatable shaft (130) slantwise.

4. Fat tissue removal device according to any of claims 1 to 3, wherein the cutting element (120) comprises at least one blade extending from the rotatable shaft (130).

5. Fat tissue removal device according to claim 4 wherein the at least one blade is arc-shaped, preferably S-shaped.

6. Fat tissue removal device according to any of the preceding claims, wherein the cutting element (120) is arranged substantially diagonally with respect to the grinding chamber (160) such that during use it occupies the majority of the grinding chamber volume.

7. Fat tissue removal device according to any of the preceding claims wherein the rotatable shaft (130) is off-centred with respect to the centre of the grinding chamber (160), towards the opening.

8. Fat tissue removal device according to any of the preceding claims wherein the blunt cap (110) is detachable from the cannula.

9. Fat tissue removal device according to any of the preceding claims wherein the blunt cap (110) comprises a second opening (116) located along the axis of the rotatable shaft (130) for the insertion of said rotatable shaft (130).

10. Fat tissue removal device according to any of the preceding claims wherein the width size of the blunt cap (110) is less than 20 mm, preferably less than 10 mm.

11. Fat tissue removal device according to any of the preceding claims wherein the blunt cap (110) is made of plastic or ceramic or stainless steel.

12. Fat tissue removal device according to any of the preceding claims wherein the rotatable shaft is provided inside a shaft tube (140) and/or is provided inside the evacuation tube (150), and/or further comprising a housing (180) configured to enclose at least partly the shaft tube (140) and the evacuation tube (150).

13. Fat tissue removal assembly comprising:
- a fat tissue removal device according to any of the preceding claims;
- an evacuation system comprising an evacuation conduit and a vacuum pump, said evacuation conduit being connected to said fat tissue removal device and being configured to evacuate fat tissue.

14. A kit of parts for the removal of fat tissue comprising:
- a blunt cap (110) configured to be fixed to a cannula (100) comprising an evacuation tube (150) connectable to an evacuation system and
- a rotatable shaft (130) comprising a cutting element (120) attached to the rotatable shaft (130), said rotatable shaft being configured to be driven by a driving device;
wherein the blunt cap (110) comprises a grinding chamber (160) for grinding fat tissue with the cutting element (120);
wherein the grinding chamber is formed by internal walls of the blunt cap (110) and by an opening (115) configured to allow entry of fat tissue inside the grinding chamber;
wherein the grinding chamber is further arranged to receive the rotatable shaft such that a portion of at least one tip of the cutting element (120) extends out of said grinding chamber (160) through said opening when in use in order to collect fat tissue inside said grinding chamber; and
wherein the grinding chamber of the blunt cap (110) is further configured to be connected to the evacuation tube to evacuate grinded fat tissue.

15. Method for the removal of fat tissue comprising the steps of:
- inserting the cannula (100) of the fat tissue removal device according to any of claims 1 to 12 or of the fat tissue removal assembly according to claim 13 in a fat tissue region of a body;
- activating the rotatable shaft (130) of said fat tissue removal device and optionally activating the evacuation device;
- grabbing fat tissue from the fat tissue region of the body through the opening (115) of the blunt cap (110) of the cannula (100);
- cutting said fat tissue in the grinding chamber (160) of said fat tissue removal device with the cutting element;
- removing said grinded fat tissue through the evacuation tube (150).

16. Method of retrofitting a device for removing fat tissue comprising a cannula, the method comprising the steps of providing the blunt cap and the rotatable shaft according to claim 14 to the cannula of the device.
